# EUROPEAN PATENT APPLICATION

(11) **EP 4 789 610 A1**
(43) Date of publication of application: **12.08.2026**
(21) Application number: 26156631.9
(22) Date of filing: 05.02.2026
(51) Int. Cl.: A61B 5/024, A61B 5/00

(54) **MEDICAL DEVICE MARKERS FOR VITAL SIGNS MONITORING**

(30) Priority: 07.02.2025 US 202563755757 P
(71) Applicant: Welch Allyn, Inc., Skaneateles Falls, NY 13153-0220 (US)
(72) Inventor: MCSWEENEY, WonKyung, Skaneateles Falls, 13153-0220 (US); LANE, John A., Skaneateles Falls, 13153-0220 (US); QUINN, David E., Skaneateles Falls, 13153-0220 (US); WOLFE, Gene J., Skaneateles Falls, 13153-0220 (US); MARTIN, Scott Andrew, Skaneateles Falls, 13153-0220 (US); POLSHIKOVA, Anzhelika, Skaneateles Falls, 13153-0220 (US); HOLTZ, Michael J., Skaneateles Falls, 13153-0220 (US)
(74) Representative: Reddie & Grose LLP

(57) **Abstract**

An example system for monitoring heart rate can include: one or more processors; and storage media encoding instructions which, when executed by the processors, causes the system to: receive video data of a patient and a medical marker positioned on a skin of the patient, wherein the medical marker comprises a material configured to mimic optical properties of the skin; detect the skin of the patient within the video data to establish a monitoring region; detect the medical marker within the monitoring region; identify a region of interest on the skin within the monitoring region; generate a first photoplethysmography (PPG) signal from the region of interest and a second signal from the medical marker; minimize environmental noise from the first PPG signal using the second signal to form a modified signal; and generate a heart rate measurement based on the modified signal.

## Description

Remote photoplethysmography (PPG) has emerged as a non-contact method for measuring vital signs of a patient. For instance, heart activity can be measured by using cameras or sensors to detect subtle changes in skin color caused by blood flow through vessels. While this technology offers the advantage of measuring vital signs without physical contact with the patient, its accuracy has been significantly limited by its sensitivity to environmental factors. Traditional video-based vital sign monitoring systems are particularly vulnerable to lighting changes, patient movement, and other environmental disturbances that can alter the light levels on the patient's skin, making continuous measurements challenging in dynamic healthcare environments.

Previous attempts to address these environmental interference challenges have included using reference regions like patient clothing or background areas, as well as implementing various sensor combinations and selective illumination techniques. However, these approaches have been limited by their reliance on multiple sensor inputs, fixed placement requirements, or the need for active electronic components. Additionally, existing solutions often struggle to maintain accuracy during patient movement or when lighting conditions fluctuate, making them less reliable for continuous monitoring in real-world clinical settings.

Examples provided herein are directed to medical device markers for vital signs monitoring.

According to one aspect, an example system for monitoring heart rate can include: one or more processors; and non-transitory computer-readable storage media encoding instructions which, when executed by the one or more processors, causes the system to: receive video data of a patient and a medical marker positioned on a skin of the patient, wherein the medical marker comprises a material configured to mimic optical properties of the skin; detect the skin of the patient within the video data to establish a monitoring region; detect the medical marker within the monitoring region; identify a region of interest on the skin within the monitoring region; generate a first photoplethysmography (PPG) signal from the region of interest and a second signal from the medical marker; minimize environmental noise from the first PPG signal using the second signal to form a modified signal; and generate a heart rate measurement based on the modified signal.

According to another aspect, an example method for monitoring heart rate can include: receiving video data of a patient and a medical marker positioned on a skin of the patient, wherein the medical marker comprises a material configured to mimic optical properties of the skin; detecting the skin of the patient within the video data to establish a monitoring region; tracking the medical marker within the monitoring region; identifying a region of interest on the skin within the monitoring region; generating a first photoplethysmography (PPG) signal from the region of interest and a second signal from the medical marker; minimizing environmental noise from the first PPG signal using the second signal to form a modified signal; and generating a heart rate measurement based on the modified signal.

The invention will now be further described by way of example with reference to the accompanying drawings, in which:
Figure 1 shows an example of a system for using medical device markers for vital signs monitoring.
Figure 2 shows an example of a patient including a medical device marker of the system of Figure 1.
Figure 3 shows an example of a medical device marker of the system of Figure 1.
Figure 4 shows another example of a medical device marker of the system of Figure 1.
Figure 5 shows another example of a medical device marker of the system of Figure 1.
Figure 6 shows another example of a patient including the medical device marker of Figure 5.
Figure 7 shows another example of a medical device marker of the system of Figure 1.
Figure 8 shows an example of logical components of a medical device of the system of Figure 1.
Figure 9 shows an example of a simulated PPG signal as captured by the medical device of Figure 8.
Figure 10 shows another example of a simulated PPG signal with noise as captured by the medical device of Figure 8.
Figure 11 shows an example of a power spectrum density plot of the PPG signal of Figure 10.
Figure 12 shows the PPG signal of Figure 10 with the noise minimized.
Figure 13 shows an example of a power spectrum density plot of the PPG signal of Figure 12.
Figure 14 shows an example of a method as implemented by the medical device of Figure 8.
Figure 15 shows example physical components of the medical device of Figure 8.

This disclosure relates to medical device markers for vital signs monitoring.

Various examples provided herein can employ a medical device marker constructed from specific materials and/or pigments that mimic human skin optical properties (e.g., using biomimicry), enabling accurate environmental noise measurement. Detection algorithms can be used that combine face detection with corner tracking to precisely locate and monitor both the marker and skin regions of interest. Through signal processing, simultaneous signals can be captured from a patient's skin and a medical marker. This approach can be used to minimize environmental noise by subtracting the marker signal from the skin signal, resulting in a cleaner vital signs measurement.

In the examples below, the described vital sign measured is heart rate. However, in alternative embodiments, many other types of vital signs can be measured. Such vital signs include, without limitation, heart rate variability, blood pressure, and/or blood oxygen saturation.

More specifically, the examples described herein provide a solution for accurate remote vital sign (e.g., heart rate) monitoring through an optical biomimicry approach. One embodiment employs a specially-constructed marker that mimics human skin's optical properties to measure environmental noise near the region of interest on a patient. This marker can be constructed using specific silicone casting methods and selected pigments to replicate skin texture and color while inhibiting, blocking out, or otherwise minimizing the visibility of blood volume changes underneath the marker on the skin, enabling it to isolate and capture only environmental noise factors like lighting changes and movement.

An example process that is employed combines detection algorithms with advanced signal processing techniques. The example process begins with face detection to establish a monitoring region, followed by precise marker detection and tracking (e.g., using corner detection algorithms and Lucas-Kanade optical flow techniques). The process can simultaneously capture signals from both the patient's skin and the medical marker, then employ a linear combination approach to eliminate environmental noise by subtracting the marker signal from the skin signal. This results in a significantly cleaner heart rate measurement that is more resistant to environmental interference.

Some advantages of these examples include its passive, external camera-based approach that allows for flexible marker placement and comprehensive noise elimination without requiring electronic components within the marker. The marker can be positioned separately from the patient and customized to match specific patient characteristics, including texture, color, and even three-dimensional (3D) scanned skin features. For instance, the marker could be placed in other positions, such as on the bedrail and/or wall. This configuration can address sensitivity to various sources of noise, such as lighting changes, patient movement, and other environmental disturbances that can impact accurate continuous measurements in dynamic healthcare environments.

There can be various advantages associated with the technologies described herein. For instance, the examples can provide a medical device such as the markers that are a concrete technological solution to the specific problem of inaccurate remote heart rate monitoring in dynamic healthcare environments. In these examples, the improvement can be directly correlated to the functionality of remote PPG technology used to estimate heart rate. The examples can be used to process real-time video data through a specific sequence of technical steps, including face detection, marker tracking, and/or signal processing, to generate more accurate heart rate measurements that are particularly valuable in clinical settings where traditional contact-based monitoring may be impractical or undesirable.

The concepts practical implementations include specific hardware and software components working together to capture simultaneous signals from both the patient's skin and a physically-constructed medical marker. This can be enhanced through the advantageous use of mathematical algorithms to eliminate environmental noise through linear combination of these signals, resulting in significantly improved heart rate measurements that enable better patient monitoring in real-world healthcare scenarios. These technological improvements address fundamental limitations of existing systems and provide tangible benefits in patient care by enabling more reliable non-contact vital sign monitoring.

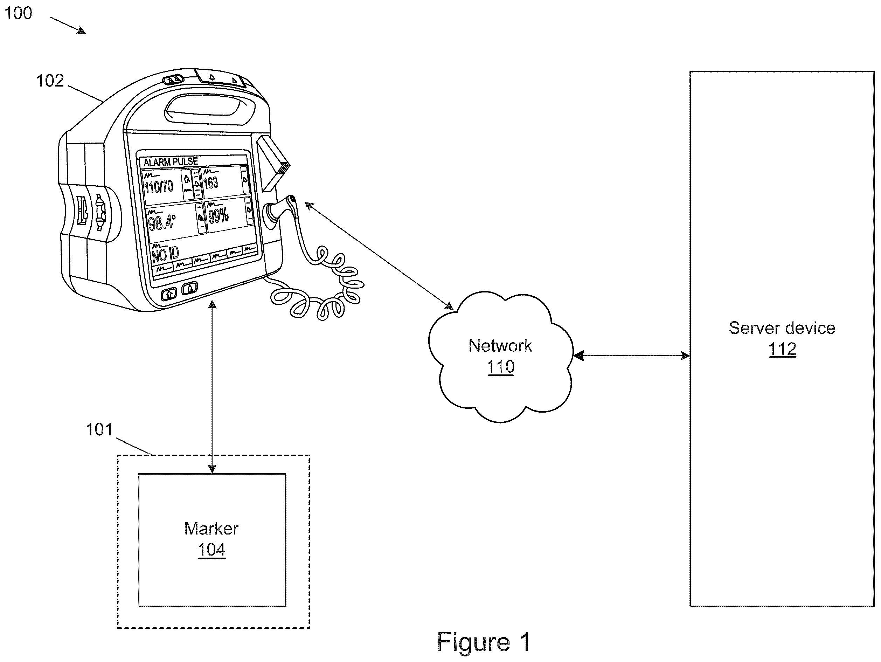

Figure 1 schematically shows aspects of an example of a system 100 programmed to use medical device markers for heart rate monitoring. In this example, the system 100 can be a computing environment that includes a plurality of client and server devices. In this instance, the system 100 includes a medical device 102 and a server device 112. The medical device 102 can communicate with the server device 112 through a network 110 to accomplish the functionality described herein.

Each of the devices may be implemented as one or more computing devices with at least one processor and memory. Example computing devices include a mobile computer, a desktop computer, a server computer, or other computing device or devices such as a server farm or cloud computing used to generate or receive data.

The example medical device 102 is programmed to monitor a patient 101. For instance, the medical device 102 may be located in a room where the patient 101 is located, such as in a caregiving facility like a clinic or hospital. The medical device 102 can also be located at the home, office, or any other place where the patient 101 is located.

Generally, the example medical device 102 is programmed to monitor one or more vital signs associated with the patient 101. Examples of such vital signs include, without limitation, temperature, blood pressure, oxygen saturation, etc.

In one example, the medical device 102 can be further configured to monitor a heart rate of the patient 101. In this embodiment, the medical device 102 includes one or more cameras positioned to capture images and/or video of the patient 101. The medical device 102 is programmed to use the images and/or video to monitor the heart rate through remote PPG. As described further herein, the medical device 102 can be programmed to use a marker 104 associated with the patient 101 to remotely monitor the heart rate. More specifically, this marker 104 can be placed near the region of interest (e.g., a cheek of the patient 101) to capture environmental noise factors like lighting changes and movement, allowing the system 100 to subtract this noise from the PPG signal obtained by the medical device 102 to provide a cleaner heart rate measurement.

In one example, the medical device 102 can be a Connex Vital Signs Monitor manufactured by Welch Allyn Inc. Example functionality of such a device is provided in U.S. Patent No. 9,265,429 filed on March 31, 2010, the entirety of which is hereby incorporated by reference. Many other configurations are possible.

The medical device 102, which can include one or more cameras and/or be programmed to interface with one or more external cameras, can be programmed to communicate the heart rate to a server device 112. The example server device 112 is programmed to store and/or present the heart rate to caregivers. For instance, in one embodiment, the server device 112 is associated with a call station for caregivers, and the server device 112 can display the heart rate. In another example, the server device 112 is associated with an electronic medical records (EMR) system that stores information associated with the heart rate in a record associated with the patient 101.

In other embodiments, the server device 112 can be programmed to process or further process the data collected by the medical device 102. For instance, in an alternative design, the medical device 102 can forward raw PPG data captured by the medical device 102, and the server device 112 can be programmed to estimate the heart rate of the patient 101 such as by using the logic described below.

The network 110 provides a wired and/or wireless connection between the medical device 102 and the server device 112. In some examples, the network 110 can be a local area network, a wide area network, the Internet, or a mixture thereof. Many different communication protocols can be used. Although only three devices are shown, the system 100 can accommodate additional types and quantities of computing devices.

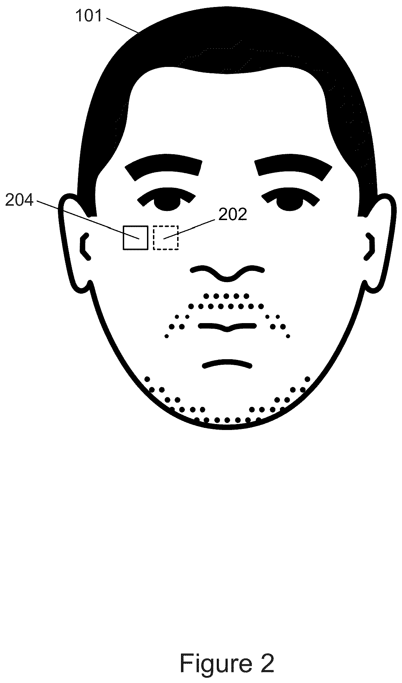

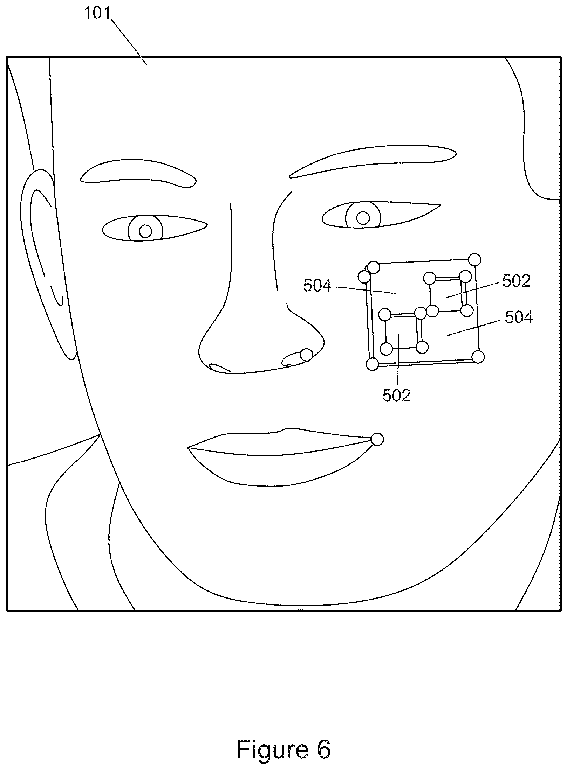

Referring now to Figures 2-7, example markers, such as the marker 104, can be configured and positioned in various manners.

Various processes can be used to create the marker 104. In these examples, the marker 104 is constructed to mimic the optical properties of the skin of the patient 101. The marker 104 can also be configured to inhibit, hide, block out, and/or otherwise hide noise associated with skin coloration changes due to arterial blood volume changes. For instance, by placing the marker 104 on the skin of the patient 101, the marker 104 shown in the video captured of the skin of the patient 101 will cover or otherwise hide the skin coloration changes under the marker 104.

For instance, a mold design and creation process for the markers can be implemented through various digital modeling approaches and software platforms. While one embodiment utilizes Blender, an open-source 3D modeling software from the Blender Foundation, other suitable computer-aided design (CAD) software packages capable of creating 3D models may be employed. The base shape can be configured as a trapezoidal form or alternatively may comprise other geometric configurations that facilitate demolding, including but not limited to pyramidal, conical, or modified rectangular shapes.

The digital model construction can begin with generating a base form, which in some implementations comprises a cubic structure that is subsequently scaled to desired dimensions. The surfaces of this base form undergo subdivision processing to create a mesh pattern, which may include uniform or non-uniform grid arrangements of varying densities. This mesh density can be adjusted based on the desired resolution of the final texture details, with higher density patterns generally enabling more precise replication of fine surface features. Alternative surface preparation techniques may include direct mesh manipulation, displacement mapping, or other digital surface modification methods.

The texture application phase can utilize various digital manipulation techniques to achieve the desired surface characteristics. While Boolean operations represent one implementation method for subtracting the skin texture pattern from the base form, alternative approaches may include displacement mapping, sculpting tools, or parametric surface modifications. The resulting negative mold may capture various topographical features including, but not limited to, skin texture patterns, pore structures, fine lines, and other surface characteristics that contribute to the optical properties of human skin. The mold design may optionally incorporate additional features such as alignment markers, registration points, or auxiliary structures to facilitate the subsequent casting process.

The digital model may be customized to replicate specific patient characteristics through various input methods, including 3D scanning of actual skin surfaces, parametric texture generation, or modification of predefined texture patterns. The mold design can also accommodate various sizes and configurations, including but not limited to single or multiple cavity designs, modular arrangements, or integrated grid patterns for enhanced tracking capabilities.

In some examples, the mold printing process can be implemented using various additive manufacturing technologies capable of producing high-precision components. In one embodiment, the mold is fabricated using a Stratasys J850 3D printer manufactured by Stratasys Ltd., selected for its ability to achieve precise dimensional control and surface finish characteristics. However, other suitable 3D printing platforms with comparable precision capabilities may be employed, including but not limited to stereolithography (SLA), digital light processing (DLP), or other photopolymer-based printing systems.

The printing material selection plays a role in achieving the desired mold characteristics. Vero photopolymer resin from Stratasys Ltd. represents one implementation due to its fine detail reproduction capabilities, high dimensional stability, and smooth surface characteristics. However, alternative materials may be selected based on specific requirements. Suitable materials may include other photopolymer resins, thermoplastic materials, or composite printing materials that demonstrate appropriate physical properties for mold applications, particularly those that enable accurate replication of human skin's biomechanical characteristics.

The printing process parameters may be optimized to achieve the desired surface quality and dimensional accuracy. These parameters can include, but are not limited to, layer thickness, exposure time, build orientation, support structure design, and post-processing requirements. The mold may optionally incorporate various surface treatments or finishing processes to enhance its performance characteristics, such as surface smoothing, coating applications, or other modifications that improve demolding properties while maintaining precise feature reproduction.

The printed mold structure may be configured to include additional functional features beyond the basic skin texture replication. These may include registration marks, alignment features, pour gates, venting channels, or other auxiliary structures that facilitate the subsequent casting process. The mold design and printing process can be adapted to accommodate various sizes, shapes, and complexity levels, enabling the production of both single-use and reusable mold configurations.

Once the mold is finished, various materials can be used to cast the markers. The casting process can be implemented using various silicone-based materials and techniques. In one embodiment, the process utilizes Dragon Skin^{™} 20, a platinum-cured silicone compound from Smooth-On, Inc., which is prepared by combining equal proportions of parts 1A and 1B of the compound by either volume or weight measurements. While Dragon Skin^{™} 20 represents one implementation due to its physical properties and flexibility, alternative silicone materials may be employed, including but not limited to Ecoflex^{™} (also from Smooth-On, Inc.), other variants in the Dragon Skin^{™} series, polydimethylsiloxane (PDMS), Mold Max^{™} (also from Smooth-On, Inc.), or comparable silicone rubber compounds that demonstrate suitable physical characteristics.

The mold preparation process includes the application of release agents to facilitate demolding. While Ease Release^{™} 200 (also from Smooth-On, Inc.) represents one implementation with a 30-minute drying period, alternative release agents or surface treatments may be employed based on compatibility with the selected casting materials and desired surface characteristics. The marker's optical properties are achieved through the incorporation of various colorants and additives. These may include, but are not limited to, silicone-compatible pigments (such as "Flesh" pigments, Silc Pig^{™}, Psycho Paint^{™}, Skin Tite^{™}, or Cast Magic^{™} powders from Smooth-On, Inc.), acrylic dyes, and fiber materials like soft flock rayon. The loading proportions of these additives typically range from 0.001% to 3% of the total silicone system weight, though these proportions may be adjusted based on specific requirements for matching patient skin characteristics.

To encourage material integrity and optimal performance characteristics, the casting mixture undergoes a degassing process to remove entrapped air. While one implementation utilizes vacuum degassing at a minimum of 29 inches of mercury (equivalent to 1 Bar or 100 KPa), alternative degassing methods or pressure levels may be employed based on material requirements and available equipment. The curing process can be conducted under various environmental conditions, with one implementation allowing the silicone to cure at room temperature (approximately 73°F/23°C) for a minimum of six hours to develop optimal elastomeric properties. The curing process may be modified through temperature adjustment or other environmental factors to accelerate or optimize the development of desired material properties.

The casting process may optionally incorporate additional steps or modifications to enhance marker performance, including but not limited to layered casting techniques, embedded structural elements, surface treatments, or the integration of tracking features. The process can be adapted to accommodate various marker sizes, shapes, and complexity levels, enabling customization for specific patient characteristics or monitoring requirements.

For instance, in an alternative process for making the markers, a negative mold is employed. This alternative process utilizes readily available dental and casting materials to achieve high-fidelity skin texture replication. The process begins with the preparation of an alginate mixture, combining the alginate material with water in a 1:1 ratio. This mixture is then applied directly to the desired patient skin area to capture detailed topographical features. While alginate represents one implementation, other suitable impression materials may be employed based on specific requirements for detail capture and working time.

The process continues with the creation of a positive replica using polyurethane rubber. The alginate negative mold is first secured within a rigid housing structure to maintain its shape during the casting process. The polyurethane mixture is then prepared and poured into the alginate mold. To ensure optimal detail reproduction, the mixture undergoes a degassing process to eliminate air bubbles that could compromise the quality of the replication. Alternative casting materials with suitable physical properties may be selected based on specific requirements for detail reproduction and durability.

The master mold creation involves treating the positive polyurethane replica with a release agent, such as Ease Release^{™} 200, allowing sufficient drying time of approximately 30 minutes. The treated positive is then positioned within a larger housing structure, creating space for an additional layer of polyurethane. This layer should maintain a minimum thickness of 10 millimeters over the entire surface to ensure structural integrity. The polyurethane mixture undergoes degassing to prevent imperfections, and the curing process follows the manufacturer's specified parameters. Alternative release agents and housing configurations may be employed based on material compatibility and specific requirements.

The final stage involves utilizing the master mold for casting the biomarker using silicone materials such as Dragon Skin^{™}, following the previously described casting processes. This method provides a practical alternative for fabricating biomarkers without requiring advanced 3D printing technology, while still achieving high fidelity in replicating human skin textures. The process can be modified to accommodate various skin textures, marker sizes, and specific patient characteristics through adjustments to the initial impression-taking process and subsequent casting steps.

Various other materials can also be used to form the markers. Nonexclusive examples of such materials include: gelatinous substances like gelatine, agar and agarose, collagens, and polyvinyl alcohol gels; elastomers like silicone (mentioned) and polyurethanes; and/or epoxy resins. Other additives can also be used to tune the desired optical properties of the markers. Nonexclusive examples of such additives include nano- and micro-particles incorporated as scatterers into solid or liquid material such as metallic gold, titanium dioxide, silicon dioxide, aluminum oxide, polystyrene, carbon black, graphite, and lipid. Many other configurations are possible.

Once the markers are formed, they can be placed on the patient 101 and used to perform remote PPG as follows.

Referring now to Figure 2, an example marker 204 is positioned on the cheek of the patient 101 near a region of interest 202. As described further herein, the medical device 102 is configured to capture images and/or video of the marker 204 and the region of interest 202 and use these images and/or video to estimate the heart rate of the patient 101.

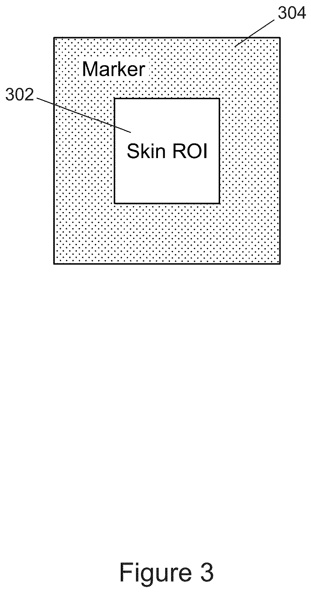

In another configuration shown in Figure 3, an example marker 304 includes an opening exposing a region of interest 302 of the skin of the patient 101. Similar to the above, the medical device 102 is configured to capture images and/or video of the marker 304 and the region of interest 302 and use these images and/or video to estimate the heart rate of the patient 101.

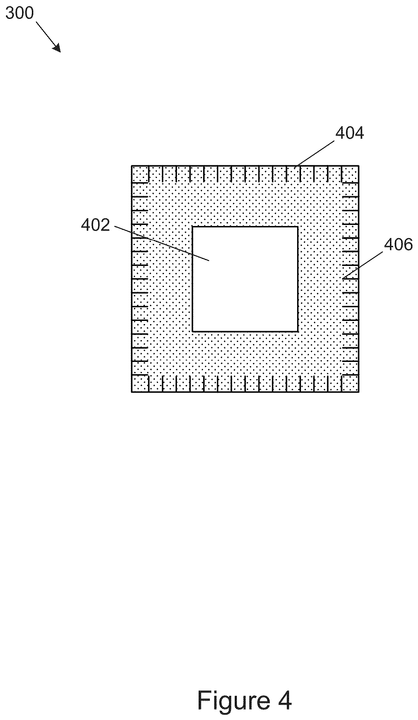

In Figure 4, an example marker 404 is configured similarly to the marker 304 to include an opening exposing a region of interest 402 of the skin of the patient 101. However, the marker 404 also include grids 406 on the marker 404 that assists the medical device 102 in locating the marker 404 and/or the region of interest 402. Similar to the above, the medical device 102 is configured to capture images and/or video of the marker 404 and the region of interest 402 (e.g., utilizing the grids 406) and use these images and/or video to estimate the heart rate of the patient 101.

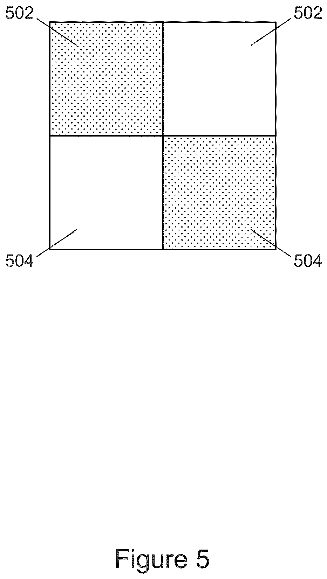

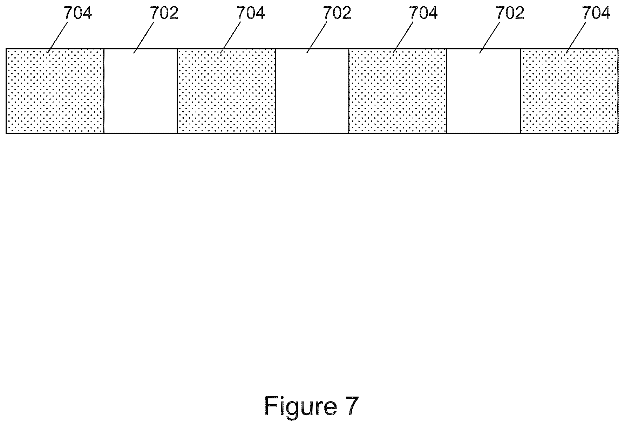

Referring to Figures 5-6, another example includes multiple markers 504 positioned adjacent to multiple regions of interest 502 of the skin of the patient 101. Similarly, in Figure 7, an example is shown with multiple markers 704 positioned linearly between multiple regions of interest 702 of the skin of the patient 101. Many other such configurations, including other patterns, can be used.

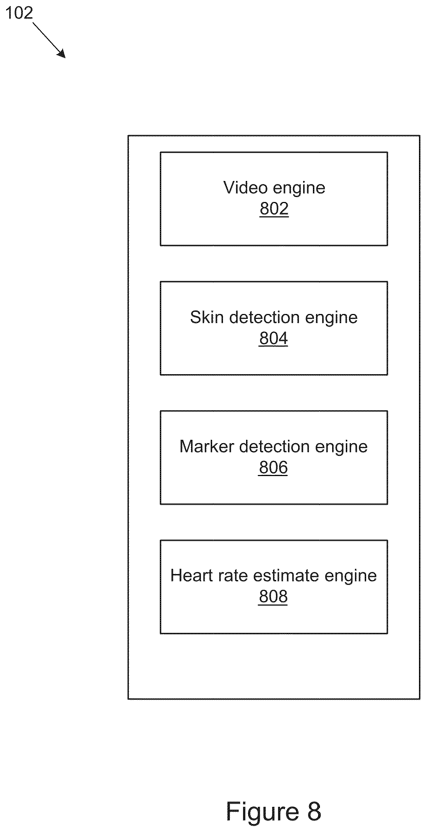

Referring now to Figure 8, additional details of the medical device 102 are shown. In this example, the server device 112 has various logical engines that assist in using the medical device markers for heart rate monitoring. The medical device 102 can, in this instance, include the following engines. In other examples, more or fewer engines providing different functionality can be used. Further, in alternate designs, one or more of the engines can be executed on the server device 112 in a similar manner.

An example video engine 802 of the medical device 102 is programmed to obtain one or more images or video of the patient 101. In this example, the video can be obtained using one or more cameras positioned on the patient 101. Such camera or cameras can be incorporated as part of the medical device 102 or as standalone units.

More specifically, various camera configurations can be used to capture PPG signals from the patient and signals the medical marker. In one implementation, a single camera is used to simultaneously monitor the region of interest on the patient's skin (typically the cheek or forehead) and the medical marker positioned nearby. Alternative configurations may include multiple cameras for enhanced tracking capabilities, cameras with specific wavelength sensitivities optimized for signal detection, or camera systems integrated with existing clinical monitoring equipment.

The camera positioning can be flexible, allowing for various mounting options and distances from the patient, while maintaining the ability to capture both the patient's region of interest and the marker within the same field of view. The video engine 802 can accommodate different camera resolutions and frame rates, provided they are sufficient to detect the subtle color changes associated with blood volume variations and environmental lighting changes captured by the medical marker.

The video engine 802 can be programmed to store the video captured of the patient 101 for processing, as described further below.

Example skin detection engine 804 and marker detection engine 806 utilize the video captured by the video engine 802 to detect the marker 104 and skin of the patient 101.

The skin detection engine 804 and the marker detection engine 806 can employ a multi-stage approach that begins with face detection to establish the initial region of analysis. The skin detection engine 804 first identifies a face within a frame of the video and creates a bounding box, which serves as the primary search area for subsequent detection steps. While face detection represents one implementation approach, alternative methods for establishing the initial region of interest may be employed based on specific monitoring requirements.

The marker detection engine 806 can then use a corner detection process involving multiple stages to ensure accurate marker identification. Initially, a preliminary corner detection algorithm analyzes the grayscale image within the face region, searching for light and dark areas separated by sharp lines. The marker detection engine 806 is configured to identify more corners than expected in the marker to ensure comprehensive detection. These preliminary corners undergo filtering processes, including the removal of outliers that deviate more than two standard deviations from the mean corner location.

In one example, the marker detection engine 806 uses a density-based spatial clustering of applications with noise (DBSCAN) algorithm to cluster the remaining corners based on density, effectively isolating the marker corners from other detected features. Alternative clustering algorithms or detection methods may be implemented based on specific requirements for accuracy and processing efficiency.

Following initial detection, the marker detection engine 806 defines a rectangular region containing the filtered corners with additional buffer space. This region undergoes secondary corner detection processing to identify the precise number of corners known to be present on the marker. The marker detection engine 806 then implements Lucas-Kanade optical flow tracking to monitor corner positions across subsequent frames, though alternative tracking algorithms may be employed. The tracked corners are used to define a polygon that establishes the outer boundaries of the marker, creating a mask to differentiate between pixels inside and outside the region of interest.

The skin detection engine 804 operates in conjunction with the marker detection engine 806 through segmentation analysis. The skin detection engine 804 processes the rectangular region to separate it into segments based on brightness characteristics in grayscale, with the brighter segments typically corresponding to skin regions. The final determination of skin and marker regions combines the polygon masking with segmentation results, where skin areas are defined as pixels within the polygon that meet the skin brightness criteria, while marker areas comprise the remaining pixels within the polygon boundaries.

Alternative segmentation approaches or classification criteria may be implemented based on specific monitoring requirements or environmental conditions. The examples can optionally utilize grid patterns on the marker edges to capture region contours and provide additional reference points for tracking and analysis.

An example heart rate estimate engine 808 is programmed to employ a noise elimination approach through linear combination of signals captured from both the skin of the patient 101 by the skin detection engine 804 and the marker 104 by the marker detection engine 806. Certain sources of noise, including breathing patterns, lighting variations, and involuntary movements, manifest similarly in both the skin and marker signals. By implementing a combination of these signals, specifically subtracting the marker signal from the skin signal, the heart rate estimate engine 808 can effectively cancel out common noise elements while preserving the desired physiological data.

More specifically, the example linear combination can be a noise elimination algorithm that combines the PPG signal from the skin with the signal from the marker by subtracting the marker signal from the skin signal. This approach assumes that certain noise sources (such as breathing, light changes, and involuntary movements) appear in both signals, so subtracting the marker signal from the skin signal allows these common noise elements to cancel out. When implemented, this linear combination algorithm: takes the skin PPG signal containing both the desired heart rate data and noise; subtracts away the marker signal which contains only environmental noise; and results in a cleaner signal where noise spikes (like those at 0.4 Hz from breathing and 0.9 Hz from neck movements) are reduced while preserving the true heart rate signal. An effectiveness of this linear combination approach is demonstrated in the simulated power spectrum density plots described further below, where after the subtraction, the spike corresponding to the true heart rate becomes the largest peak while the noise-related spikes are diminished.

The heart rate estimate engine 808 begins with the simultaneous capture of signals from both the region of interest on the patient's skin by the skin detection engine 804 and the marker by the marker detection engine 806. These signals contain various components, including the desired heart rate information along with environmental noise factors.

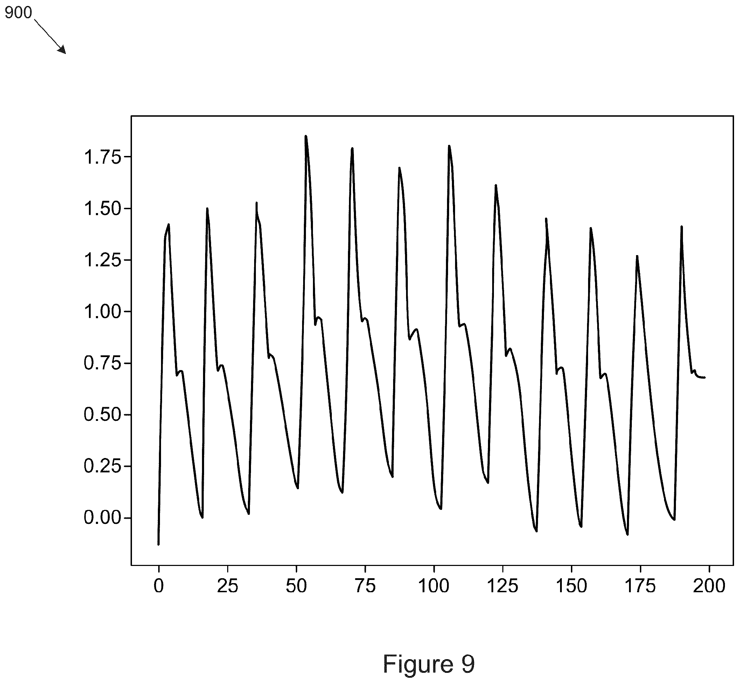

An example of such a simulated PPG signal 900 with synthetic data is shown in Figure 9, which shows a twenty second capture of a simulated PPG signal. In this example, the x-axis is time, and the y-axis is an amplitude of the signal PPG signal 900.

In this example, the PPG signal 900 represents a 70 beats per minute heart rate. However, as noted, a PPG signal can include various extraneous noise that makes it difficult to obtain the heart rate.

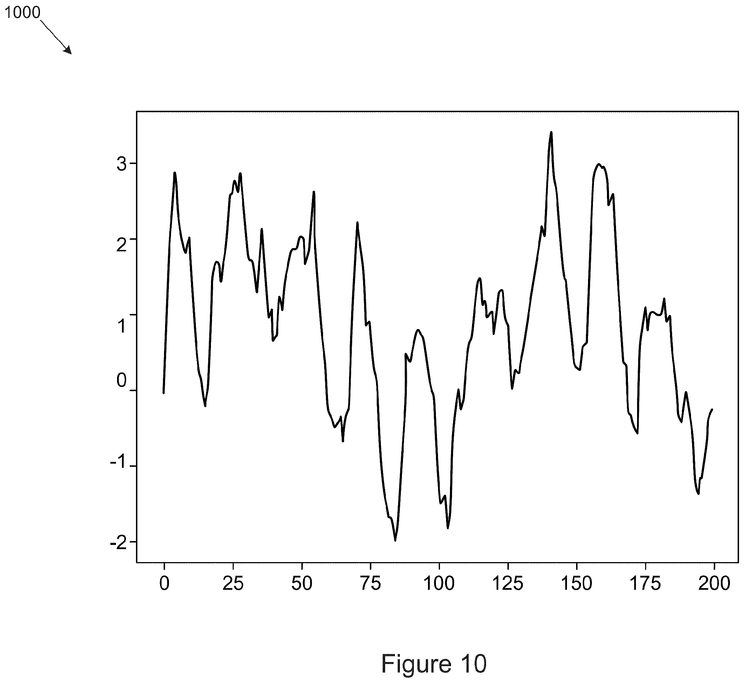

For instance, Figure 10 shows a simulated PPG signal 1000 including the PPG signal (e.g., the PPG signal 900) with added various noise (e.g., from breathing and neck movements). The heart rate estimate engine 808 processes the signal 1000 through a linear combination algorithm that identifies and isolates the common noise patterns present in both signals. This approach can effectively reduce or eliminate interference from multiple sources in the PPG signal 1000, including but not limited to breathing artifacts (typically around 0.4 Hz) and involuntary movements (such as those around 0.9 Hz).

The effectiveness of this noise elimination technique is demonstrated through power spectrum density analysis, where the combined signal processing approach significantly reduces noise-related frequency spikes while enhancing the prominence of the true heart rate signal. In experimental implementations, the heart rate estimate engine 808 has shown the ability to maintain signal clarity even in the presence of various environmental disturbances. The linear combination method can be adapted to accommodate different noise patterns and signal characteristics through adjustments to the processing parameters.

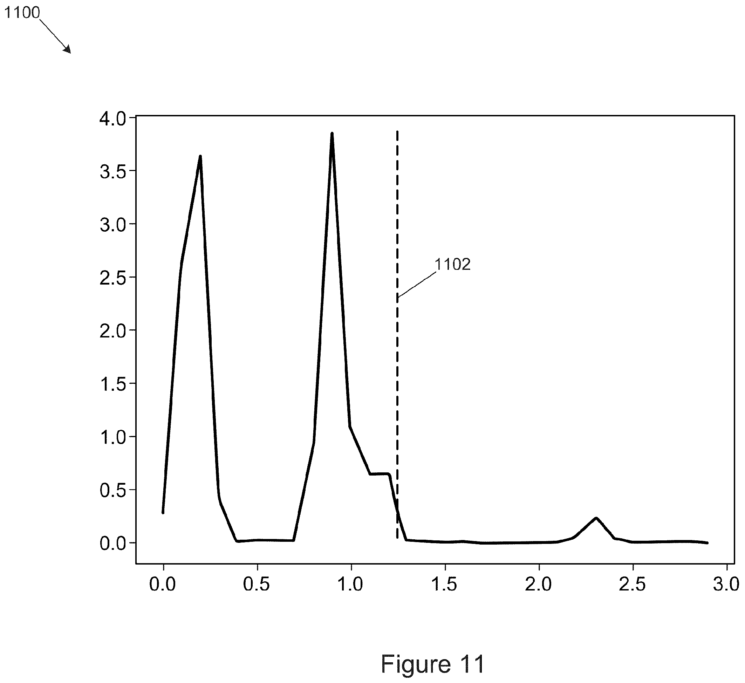

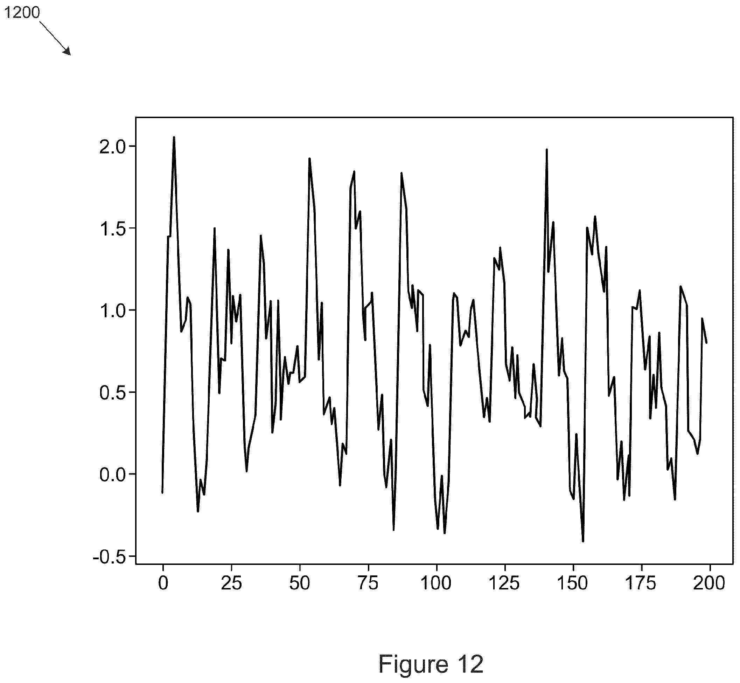

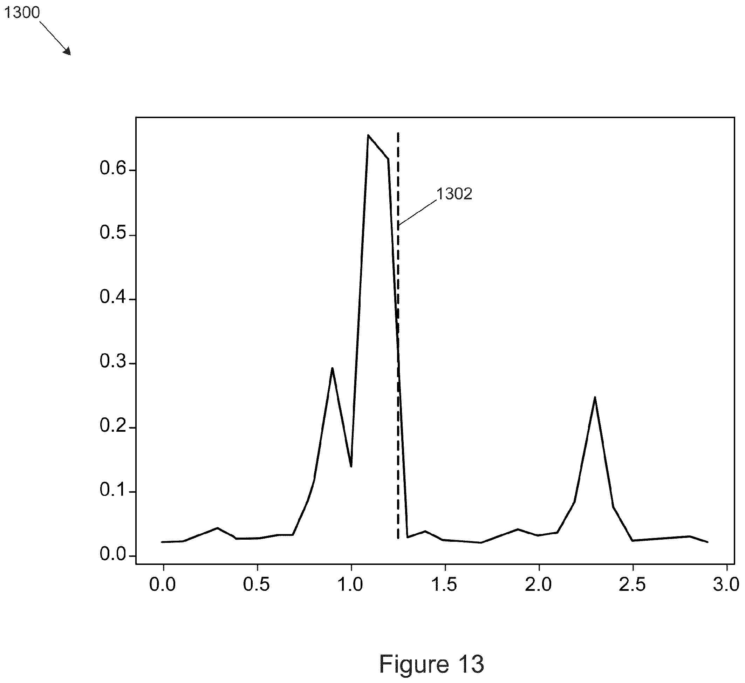

For instance, Figure 11 shows a power spectrum density plot 1100 of the signal 1000, with the x-axis being the frequency (in Hz) and the y-axis being the power amplitude (e.g., normalized spectral density). The true heart rate is marked at line 1102 (approximately 1.17 Hz) shown in the plot 1100. Figure 12 shows a simulated PPG signal 1200, with the noise as represented on the marker being minimized by the heart rate estimate engine 808. The spikes created by the noise, including breathing and neck movements, are minimized, and the correct heart rate 1302 is now shown as the largest spike, as illustrated on the power spectrum density plot 1300 shown in Figure 13.

The heart rate estimate engine 808 may optionally incorporate additional signal processing techniques to enhance noise elimination effectiveness, including but not limited to frequency domain analysis, adaptive filtering, or other mathematical approaches for signal separation and noise reduction. The processing parameters can be optimized based on specific monitoring conditions, patient characteristics, or environmental factors to achieve optimal signal quality.

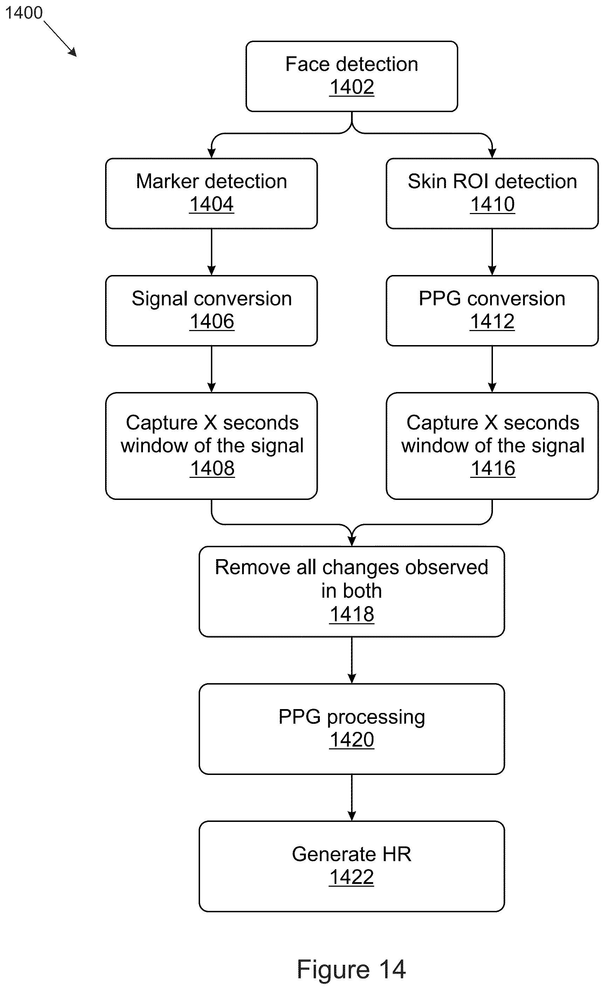

Figure 14 shows an example method 1400 as implemented by the medical device 102 of the system 100.

At step 1402, face detection is performed using computer vision algorithms to identify and isolate a face within the video frame. Step 1402 can include establishing a primary bounding box around the detected face, which serves as the initial region for subsequent processing steps. Once the face is detected and the bounding box is established, the method 1400 proceeds to locate the marker within this defined region.

Building upon the established face region, step 1404 performs marker detection that can include employing a sophisticated multi-stage approach beginning with preliminary corner detection in the grayscale image. Step 1404 can include first identifying more corners than expected to ensure comprehensive coverage, then filtering outliers that deviate more than two standard deviations from the mean corner location. The DBSCAN clustering algorithm separates marker corners from other features based on density analysis. After establishing the marker boundaries, the method 1400 can simultaneously process both marker and skin regions.

Following successful marker detection, at steps 1406 and 1412, one or more conversions process the video data (typically red, green, and blue (RGB)) from both regions to extract the relevant signals. This parallel conversion analyzes the subtle color changes in both the skin and marker regions, capturing blood volume variations in the skin signal and environmental factors in the marker signal. With the initial signals extracted, the method 1400 moves to capture specific temporal segments.

For example, for the step 1412, a color space transformation to yCbCr can be performed on the RGB signal captured by the camera from the skin of the patient. The blue-difference chroma component (Cb) and the red-difference chroma component (Cr) can then be used to extract the PPG signal for the heart rate on the skin of the patient. A similar process can be used to convert the RGB signal in the step 1406 to obtain the signal from the marker. Other color spaces can also be used.

To enhance synchronized analysis, at steps 1408 and 1416, the method 1400 captures defined temporal windows of the signals, typically spanning multiple seconds to ensure sufficient data for accurate analysis. With both signal windows captured, the method 1400 can proceed with detailed skin region analysis.

Concurrent with the marker signal capture, at step 1410, skin region of interest detection utilizes segmentation analysis to identify the specific skin region for monitoring. Step 1410 can include processing the defined region using brightness-based segmentation in grayscale, typically identifying brighter segments as skin regions. Once both the skin and marker signals are properly captured and segmented, the method 1400 can begin the noise elimination process.

Utilizing the synchronized signals from both regions, at step 1418, noise elimination is performed through linear combination of the marker and skin signals. This process identifies common noise patterns, including breathing artifacts and involuntary movements, and subtracts the marker signal from the skin signal.

For instance, as described previously, the two signals can be processed. During the frequency analysis, any activity in the frequency that is also seen in the marker signal can be minimized or eliminated, similar to a band pass filter. With the environmental noise removed, the method 1400 can proceed to final signal processing.

Building on the cleaned signal, at step 1420, signal processing techniques can be used to further enhance signal quality. This refined signal that results from step 1420 can improve the accuracy of the heart rate calculation in the final step.

Finally, at step 1422, heart rate generation converts the processed PPG signal into a numerical heart rate measurement. This step analyzes the frequency components of the cleaned signal to identify the dominant frequency corresponding to the heart rate, producing the final measurement output that represents the successful completion of the entire process.

Various changes and configurations can be made to modify the method 1400 to arrive at the desire outcome.

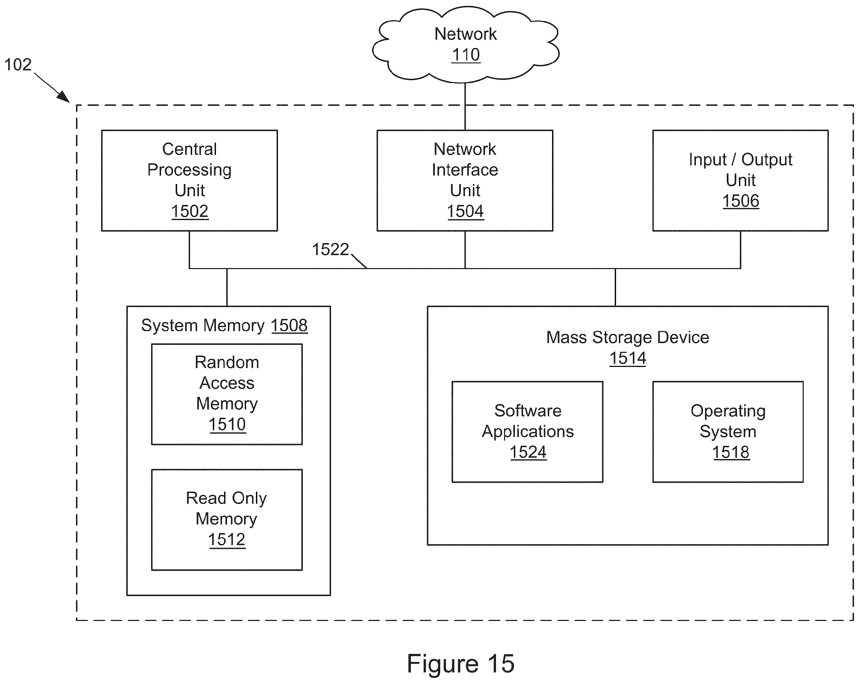

As illustrated in the embodiment of Figure 15, the example medical device 102, which provides the functionality described herein, can include at least one central processing unit ("CPU") 1502, a system memory 1508, and a system bus 1522 that couples the system memory 1508 to the CPU 1502. The system memory 1508 includes a random access memory ("RAM") 1510 and a read-only memory ("ROM") 1512. A basic input/output system containing the basic routines that help transfer information between elements within the medical device 102, such as during startup, is stored in the ROM 1512. The medical device 102 further includes a mass storage device 1514. The mass storage device 1514 can store software instructions and data. A central processing unit, system memory, and mass storage device similar to that shown can also be included in the other computing devices disclosed herein.

The mass storage device 1514 is connected to the CPU 1502 through a mass storage controller (not shown) connected to the system bus 1522. The mass storage device 1514 and its associated computer-readable data storage media provide non-volatile, non-transitory storage for the medical device 102. Although the description of computer-readable data storage media contained herein refers to a mass storage device, such as a hard disk or solid-state disk, it should be appreciated by those skilled in the art that computer-readable data storage media can be any available non-transitory, physical device, or article of manufacture from which the central display station can read data and/or instructions.

Computer-readable data storage media include volatile and non-volatile, removable, and non-removable media implemented in any method or technology for storage of information such as computer-readable software instructions, data structures, program modules, or other data. Example types of computer-readable data storage media include, but are not limited to, RAM, ROM, EPROM, EEPROM, flash memory or other solid-state memory technology, CD-ROMs, digital versatile discs ("DVDs"), other optical storage media, magnetic cassettes, magnetic tape, magnetic disk storage or other magnetic storage devices, or any other medium which can be used to store the desired information and which can be accessed by the medical device 102.

According to various embodiments of the invention, the medical device 102 may operate in a networked environment using logical connections to remote network devices through network 110, such as a wireless network, the Internet, or another type of network. The medical device 102 may connect to network 110 through a network interface unit 1504 connected to the system bus 1522. It should be appreciated that the network interface unit 1504 may also be utilized to connect to other types of networks and remote computing systems. The medical device 102 also includes an input/output controller 1506 for receiving and processing input from a number of other devices, including a touch user interface display screen or another type of input device. Similarly, the input/output controller 1506 may provide output to a touch user interface display screen or other output devices.

As mentioned briefly above, the mass storage device 1514 and the RAM 1510 of the medical device 102 can store software instructions and data. The software instructions include an operating system 1518 suitable for controlling the operation of the medical device 102. The mass storage device 1514 and/or the RAM 1510 also store software instructions and applications 1524, that when executed by the CPU 1502, cause the medical device 102 to provide the functionality of the medical device 102 discussed in this document.

Embodiments of the invention can be described with reference to the following numbered clauses, with preferred features laid out in the dependent clauses:
1. A method for monitoring heart rate, comprising:
   receiving video data of a patient and a medical marker positioned on a skin of the patient, wherein the medical marker comprises a material configured to mimic optical properties of the skin;
   detecting the skin of the patient within the video data to establish a monitoring region;
   tracking the medical marker within the monitoring region;
   identifying a region of interest on the skin within the monitoring region;
   generating a first photoplethysmography (PPG) signal from the region of interest and a second signal from the medical marker;
   minimizing environmental noise from the first PPG signal using the second signal to form a modified signal; and
   generating a heart rate measurement based on the modified signal.
2. The method of clause 11, further comprising:
   identifying corners within the monitoring region using a corner detection algorithm;
   filtering the corners using density-based spatial clustering; and
   tracking the corners as filtered across video frames using optical flow.
3. The method of clause 11, wherein the medical marker comprises a silicone compound mixed with pigments and fibers in proportions configured to match coloration of the skin of the patient.
4. The method of clause 13, wherein the silicone compound comprises a platinum-cured silicone compound mixed with the pigments in a range of 0.001% to 3% of total weight.
5. The method of clause 11, further comprising:
   capturing synchronized temporal windows of the first PPG signal and the second signal; and
   subtracting environmental noise components detected in the second signal from the first PPG signal.
6. The method of clause 11, further comprising:
   detecting a grid pattern on edges of the medical marker; and
   using the grid pattern to determine contour information for the region of interest.
7. The method of clause 11, further comprising performing brightness-based segmentation within the monitoring region to separate skin areas from non-skin areas.
8. The method of clause 11, wherein the medical marker is constructed using a mold created from a three-dimensional scan of patient skin texture.
9. The method of clause 11, further comprising analyzing frequency components of the modified signal to identify a dominant frequency corresponding to heart rate.
10. The method of clause 11, further comprising analyzing frequency components of the modified signal to perform a linear combination of the first PPG signal with the second signal to form the modified signal.

## Claims

1. A system for monitoring heart rate, comprising:
one or more processors; and
non-transitory computer-readable storage media encoding instructions which, when executed by the one or more processors, causes the system to:
receive video data of a patient and a medical marker positioned on a skin of the patient, wherein the medical marker comprises a material configured to mimic optical properties of the skin;
detect the skin of the patient within the video data to establish a monitoring region;
detect the medical marker within the monitoring region;
identify a region of interest on the skin within the monitoring region;
generate a first photoplethysmography (PPG) signal from the region of interest and a second signal from the medical marker;
minimize environmental noise from the first PPG signal using the second signal to form a modified signal; and
generate a heart rate measurement based on the modified signal.

2. The system of claim 1, wherein, to detect and to track the medical marker, the computer-readable storage media encodes further instructions which, when executed by the one or more processors, causes the system to:
identify corners within the monitoring region using a corner detection algorithm;
filter the corners using density-based spatial clustering; and
track the corners as filtered across video frames using optical flow.

3. The system of any preceding claim, wherein the medical marker comprises a silicone compound mixed with pigments and fibers in proportions configured to match coloration of the skin of the patient while inhibiting decolorization of the skin associated with blood volume changes.

4. The system of claim 3, wherein the silicone compound comprises a platinum-cured silicone compound mixed with the pigments in a range of 0.001% to 3% of total weight.

5. The system of any preceding claim, wherein, to minimize the environmental noise, the computer-readable storage media encodes further instructions which, when executed by the one or more processors, causes the system to:
capture synchronized temporal windows of the first PPG signal and the second signal; and
subtract environmental noise components detected in the second signal from the first PPG signal.

6. The system of any preceding claim, comprising further instructions which, when executed by the one or more processors, causes the system to:
detect a grid pattern on edges of the medical marker; and
use the grid pattern to determine contour information for the region of interest.

7. The system of any preceding claim, wherein, to identify the region of interest, the computer-readable storage media encodes further instructions which, when executed by the one or more processors, causes the system to perform brightness-based segmentation within the monitoring region to separate skin areas from non-skin areas.

8. The system of any preceding claim, wherein the medical marker is constructed using a mold created from a three-dimensional scan of patient skin texture.

9. The system of any preceding claim, wherein, to generate the first PPG signal, the computer-readable storage media encodes further instructions which, when executed by the one or more processors, causes the system to analyze frequency components of the modified signal to identify a dominant frequency corresponding to heart rate.

10. The system of any preceding claim, wherein, to minimize the environmental noise, the computer-readable storage media encodes further instructions which, when executed by the one or more processors, causes the system to analyze frequency components of the modified signal to perform a linear combination of the first PPG signal with the second signal to form the modified signal.
